# EUROPEAN PATENT SPECIFICATION

(11) **EP 0 277 175 B1**
(45) Date of publication and mention of the grant of the patent: **24.02.1993**
(21) Application number: 87905095.3
(22) Date of filing: 23.07.1987
(51) Int. Cl.: A61F 2/66

(54) **PROSTHETIC FOOT**
FUSSPROTHESE
PROTHESE POUR PIED

(30) Priority: 28.07.1986 US 889885
(43) Date of publication of application: 10.08.1988
(73) Proprietor: THE OHIO WILLOW WOOD COMPANY, INC., MOUNT Sterling, OH 43143 (US)
(72) Inventor: ARBOGAST, Robert, E., Mount Sterling, OH 43143 (US); ARBOGAST, C., Joseph, Mount Sterling, OH 43143 (US)
(74) Representative: UEXKÜLL & STOLBERG
(86) International application number: US8701743
(87) International publication number: WO8800815

(56) References cited:
- DE-C- 316 514
- DE-C- 354 246
- DE-C- 361 972
- DE-C- 807 214
- FR-A- 2 240 713
- GB-A- 277 760
- GB-A- 1 420 627
- GB-A- 1 563 246
- GB-A- 2 000 033
- GB-A- 2 092 451
- US-A- 1 617 926
- US-A- 2 556 525
- US-A- 3 438 587
- US-A- 3 484 871
- US-A- 3 766 569
- US-A- 3 833 941
- US-A- 4 177 525
- US-A- 4 328 594
- US-A- 4 512 038
- US-A- 4 547 913
- US-A- 4 555 817
- US-A- 4 645 509

## Description

### Background of the Invention

This invention relates to an artificial or prosthetic food as specified in the preamble of Claim 1. Such an artificial foot is known from FR-A-2 240 713. More particularly, this invention relates to a prosthetic foot which incorporates improved energy storing capabilities by using an auxiliary deflection plate. Still more particularly, this invention relates to a lightweight prosthetic foot which, with its energy storage capabilities, provides a smooth natural motion for the user. Still more particularly, this invention relates to a prosthetic foot having an improved keel, spring structure, and method of manufacture which provides a lightweight, easy-to-use, yet sturdy S.A.C.H. prosthetic foot.

A number of types of prosthetic feet are known to the art which attend to various problems relating to the structure, cosmetic appearance, weight, and energy storage and transfer characteristics of the foot. An early design of a prosthetic foot incorporated a leather hinge across the ball of the foot with natural rubber positioned in a v-groove above the hinge. In use, the rubber member was compressed to store energy for the next step of the user. Later examples of such feet utilized various elastic materials acting as springs and energy absorbers. Thus, the notion that energy storage capabilities within a prosthetic foot could be advantageous to an amputee is not new. Such a device today is unsatisfactory because of its weight at the furthest point from the knee center which creates a large moment resulting in high stresses about the knee and greater impact at full extension of the leg. Moreover, such a device does not provide the amputee with sufficient energy storage capabilities and is unacceptable cosmetically.

GB-A-2 000 033 specifies an artificial foot having a keel of wood comprising a cavity and an outer leather covering. Another prosthetic foot introduced about 1960 included an inner keel of wood surrounded by flexible foam plastic. The plastic has a sufficient density to permit limited resistance to bending, thus creating some energy storing characteristics. Later devices have improved upon this design by carving keels from solid, homogeneous plastic blocks in an effort to relieve some of the stresses on the flexible foam plastic and to allow somewhat greater energy storage characteristics. However, these solutions have not proven to be completely satisfactory because of the weight of the foot and the eventual failure of the flexible foam urethane. In U.S. Patent No. 4,177,525 an artificial foot construction is shown as having a rigid keel made from a molded plastic material with a metal reinforcing strip embedded within the plastic keel portion near the lower surface. The keel is surrounded by a typical flexible foam plastic material molded to the keel to form the outer surface of the foot. While this device has proven to be commercially acceptable, it remained a problem in the art to improve upon that artificial foot by incorporating significantly improved energy storing capabilities, and selecting materials which made the device lightweight, yet sturdy, and capable of a long commercial life.

In U.S. Patent Nos. 3,484,871 and 3,766,569 the concept of utilizing leaf springs in an artificial foot is shown. An artificial foot having a solid in-elastic core preferably made of wood extends substantially the entire height of the foot. A front core portion extends forwardly of the main core to receive an upwardly-offset, flat, spring seat for receiving a flat, elastic leaf spring made from a pair of spring plates of uniform gauge, width, and length, and arranged in a laminar form. A flexible plastic liner, preferably made of Teflon brand material, is interposed between the plates. The strength and gauge of the spring plates is such that when the foot is flexed, such as when the weight of the wearer shifts forward over the ball of the foot in walking, the spring will flex about the ball of the foot and maintain its elastic properties to restore the toe of the foot to its original position after each walking step when the weight of the wearer is released. A protective webbing material, such as a Nylon brand material, is fitted between the lower spring plate and the sole to reduce wear, while the foot is surrounded by a resilient cover.

Thus, it is an aim in this art to provide a lightweight prosthetic S.A.C.H. artificial foot made from materials and constructed to exhibit favorable walking and running characteristics. To this end, attention is particularly given in this invention to an improved keel construction, the keel material, an improved spring construction, the spring material, and to a method of making the spring.

Notwithstanding those feet in the art, it has remained a particular problem to improve upon the energy transfer characteristics of the foot. Thus, is an overall objective of this invention to utilize an improved spring structure made from a composite material and having sufficient energy storing capability to meet the needs of the user.

It is another overall object of this invention to provide a lightweight prosthetic foot of the type described having a strong, yet lightweight, rigid compression molded composite keel.

It is still another object of this invention to provide a prosthetic foot of the type described which utilizes a laminate of a fiber-reinforced, carbon composite for the spring plates.

It is still another object of this invention to provide a prosthetic foot having a primary carbon-composite deflection plate and an auxiliary carbon-composite deflection plate for improving the energy storage and release characteristics of the foot, while ensuring its light weight.

These and other objects of the invention will become apparent from the detailed written description of the invention which follows, taken in conjunction with the accompanying drawings.

### Brief Summary of the Invention

The invention relates to an artificial foot as specified in Claim 1.

The keel is manufactured from an aramid fiber-reinforced, Nylon brand, thermoplastic having high impact resistance, and little compression set, while being resistant to fracture. The keel is made from a bottom plate and a pair of opposed, upwardly-extending side walls forming hollow forwardly-and rearwardly-opened channels, for receiving a light-weight material therein, and closed at its top by an upper plate secured to the side walls.

A primary deflection plate is made from a thermoset carbon, fiber-reinforced, multiple-layer, laminate secured to a lower, forward portion of the keel. An auxiliary deflection plate is interposed between the primary deflection plate and the keel and includes a forward, upwardly-curved portion at about the area of flexure of the foot. The graphite or carbon composite material provides significantly improved energy storage and release capability over metallic springs. Preferably, the carbon, flat spring is produced with fibers oriented relatively longitudinally and transversely so that the short, forwardly extending flat spring may undergo a large amount of deflection while resisting stresses. The method of manufacture of the deflection plate by longitudinally and transversely orienting the fibers is also a significant feature of this invention.

Alternatively, the auxiliary spring member may be made from a plastic material secured to the keel, or integrally formed with and of the same material as the keel.

The spring structure is preferably surrounded with a protective sock, preferably made of a Kevlar brand material, about which a low-density, flexible thermoplastic material, such as foam urethane is molded to provide an attractive appearance for the foot. A medium density flexible foam urethane wedge is disposed beneath the heel portion of the foot.

These and other features of the invention will also become apparent from a detailed written description which follows.

### Brief Summary of the Drawings

In the drawings:
Fig. 1 is a side cross-sectional view showing the components of the improved prosthetic foot according to the invention;
Fig. 2 is a top plan portion of the foot of Fig. 1 taken along a lower portion thereof and showing the primary and auxiliary deflection plates;
Fig. 3 is a view similar to Fig. 2 showing a top view of the keel secured to the deflection plates;
Fig. 4 is another top plan view similar to Figs. 2 and 3 showing a protective sock surrounding the deflection plates;
Fig. 5 is an exploded perspective view of the keel structure according to the invention;
Fig. 6 is a side view of the laminated spring plate construction, and
Fig. 7 is a block diagram showing steps in the manufacture of the deflection plates of a composite graphite, fiber-reinforced material.

### Detailed Description of the Preferred Embodiment

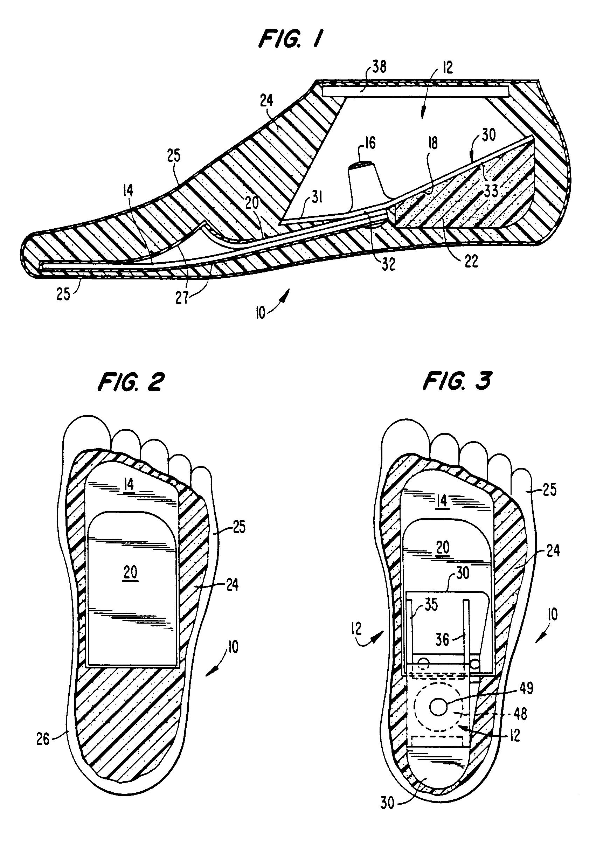

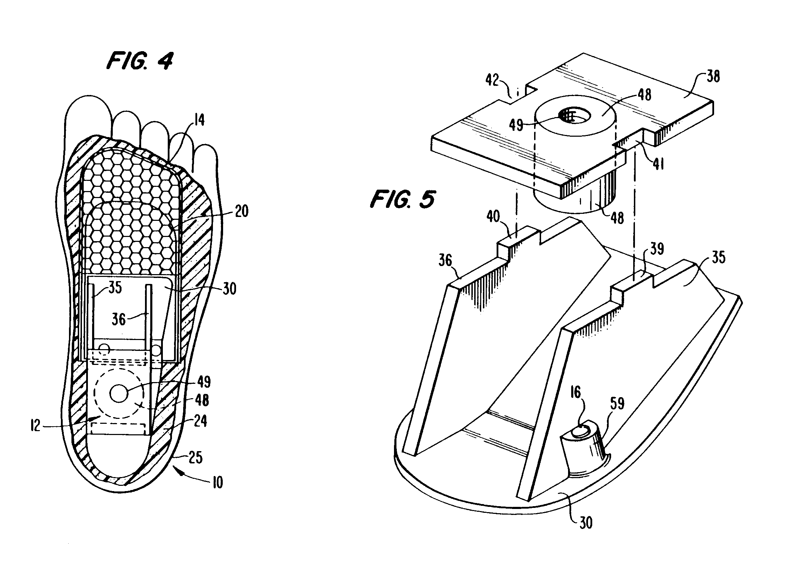

In Figs. 1-4, a prosthetic foot according to the invention is designated generally by the reference numeral 10. The foot 10 includes a rigid, compression-molded composite keel 12 to which is secured a primary carbon deflection plate 14 by a fastening member 16 to a lowermost surface 18 of a lower wall of the keel 12. An auxiliary composite deflection plate 20 is similarly secured to the surface 18 of the keel 12, intermediate the primary deflection plate 14 and the lowermost surface 18 of the keel 12. A medium-density, flexible, foam-urethane wedge 22 is located beneath the lower surface 18 of the keel 12, rearward of the fastener 16. A low density flexible foam urethane member 24 is provided about the keel and spring structure and is further surrounded by an aesthetically-appearing, cosmetically-true plastic covering 25 resembling a foot.

The keel 12 includes a laterally-extending lower wall 30 defining the lower surface 18 and having a forwardly-extending portion 31, an intermediate, upwardly-extending portion 32, and an rearwardly-extending portion 33. The angle of inclination in the rearward direction of the portion 32 is intermediate that of the forwardly-extending portion 31 and that of the rearwardly-extending portion 33.

The keel 12 further includes a pair of opposed vertically-extending walls 35, 36 spaced apart and defining a hollow channel open at its front and rear to define a receptacle for receiving a lightweight foam material 37 therein. The walls 35, 36 are joined at their uppermost top surfaces by a top plate 38 having an opening 49 therein for receiving a connection to an artificial limb structure. Thus, the walls 35 and 36 when molded provide a unitary construction with the bottom plate 30 to define a box-shaped receptacle having an irregular shape which is closed by the top plate 38. Preferably, the top member 38 has a pair of opposed, spaced recesses 41, 42 at its opposed edges for mating with upwardly-extending projections 39,40 on an uppermost surface of the side walls 35, 36.

A pair of opposed bosses 59 are molded outwardly of the side walls 35, 36 for receiving the fasteners 16 in a secure, sturdy relationship. The top plate 38 preferably includes a downwardly-extending boss 48 having a metallic insert 49 for receiving a threaded member of the prosthesis.

Preferably, the keel 12 is manufactured from an aramid, fiber-reinforced Nylon thermoplastic. These materials typically demonstrate extreme impact resistance, little compression set, and are sufficiently lightweight to reduce significantly the weight of the foot. By using this material for the keel 12, and in the hollow construction described, the keel is light-weight creating a smaller moment about the axis of the knee and thus causes significantly less impact at full extension of the prosthesis during walking or running. The structural design renders the keel 12 highly resistant to fracture, in comparison to prosthetic feet with wooden keels. The thermoplastic material also exhibits a very low compression set and water absorption characteristics, assisting in eliminating a problem of the foot becoming loose from the remaining artificial limb or prosthesis at its attachment point as is common with keels manufactured from both wood and homogeneous plastics.

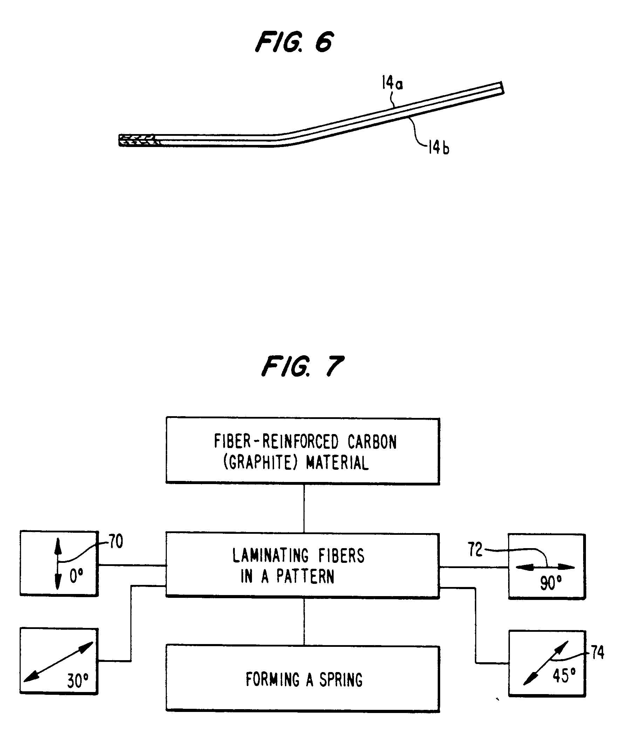

As best seen in Fig. 6 the primary deflection plate 14 is made from a pair of thin fiber-reinforced, carbon deflection plates 14a, 14b, which are slidable relative to each other about the fastener 16. The use of a thermoset carbon-reinforced multiple-spring 14 attached to the keel 12 provides significant advantages. Foremost, this material replaces the mechanical function of both a flexible, foam, plastic shell and other materials to share some of the other required load-bearing characteristics compared to some prosthetic feet known to the art. Such a design provides significant energy storage characteristics in comparison with other designs, but with a lightweight material and small required volume, permitting the grouping of the plates to be fashioned in a design that allows the amputee to more closely demonstrate the gait of a normal person.

The strength of the structure when made from a graphite or carbon composite material exhibits significant advantages over conventional heat-treated steel. From a beam analogy, a beam can be considered to be composed of an infinite number of small diameter fibers running from end to end. A beam thus supported at each end will measurably deflect when a weight is applied to the middle of the beam to cause the beam to bow downwardly. The fibers on the outermost surface of the beam, i.e., the top and bottom flanges of the beam, undergo the greatest amount of stress because their compression at the top is at a maximum while the fibers on the lowermost or bottom surface of the beam will undergo the maximum tensile forces. Thus, the fibers on the lowermost portion of the bottom flange of the beam are subjected to the greatest amount of tensile load so that the strength of these fibers determines the ultimate strength of the beam. The tensile strength of graphite composite fibers is about 1722,5 MPa (250,000 psi). while the tensile strength of steel is about 551,2 MPa (80,000 psi). This differential means that the beam can be loaded with nearly three times the load of a steel beam before permanent deformation or fracture. In addition, the graphite composite has a significantly less weight per volume compared to steel. The density of the graphite composite is about 1612 kg/cm³ (.058 lbs./cu. inch) as opposed to 839,6 kg/cm³ (0.282 lbs./cu. inch) for steel. Thus, the weight per unit volume of the graphite composite is only about 20% that of steel. Accordingly, a graphite beam will be about 15 times as resistant to deflection compared to a steel beam using the same material weights or, conversely, a graphite beam having the same resistance as a steel beam will only weigh about 1/15 as much as the steel beam. Moreover, the modulus of elasticity of the graphite composite is approximately that of the steel.

The spring is made up, as described, from a plurality of layers of unidirectional pre-preg graphite material which is commercially available with an epoxy and a removable backing. The composite is cured as is known in the art. Strips of such material are successively laminated with the fiber direction oriented as described.

The composite graphite flat spring is preferably produced with fibers running longitudinally and transversely, as shown diagrammatically in Fig. 7. When a percentage of transverse fibers are present, the composite has significant strength in the transverse direction. While this feature somewhat increases the weight of the composite flat spring 14, it remains significantly lighter. The weight of the graphite or carbon spring required to store energy in the foot 10 is approximately 80 grams of a total foot weight of 480 grams, while a steel spring to achieve the same affect would weigh approximately 580 grams of a 1080 gram foot. Since this is nearly twice the weight of any currently available prosthetic S.A.C.H. foot available, the weight is a crucial factor in the selection of the prosthetic feet to the user.

Preferably, the auxiliary deflection plate 20 is made from the same material.

As shown in Fig. 7, a certain number of transverse fibers are necessary to give the laminate strength and directions other than the longitudinal direction. By referring to the longitudinal direction as 0 degrees, as shown by the arrow 70, the composite is generally made by arranging fibers sequentially through multiple layers with the fibers oriented in the 90 and 45 degrees directions, shown by the arrows 72 and 74. Only a very few arrangements of fibers are needed to render the composite with sufficient strength without additional useless weight. Preferably, 40% of the fibers run in the 0 degree direction, while 40% run in the 30 degree direction, shown by arrow 65, and 20% run in the 90 degree direction.

The auxiliary composite deflection plate 20 has a first portion extending in a relatively planar direction in contact with a corresponding portion of the primary deflection plate 14. The auxiliary composite deflection plate 20 preferably terminates an upwardly extending arcuate portion, located at about the natural area of transverse flexion of the foot. While the auxiliary plate is preferably made as a separate component of fiber-reinforced graphite or carbon as described, alternatively it may be made of a plastic material to provide auxiliary spring action. Or, it may be made integrally with the keel of the same or different material as the keel.

## Claims

1. An artificial foot having a keel portion (12) comprising a compression molded composite keel formed to define a cavity having a lower wall, a pair or upwardly extending opposed side walls (35, 36), merging with said lower wall to define therebetween a cavity, a cushioned heel (22), spring means (14) secured to the keel for providing energy absorption and release during flexion of the foot by a user **characterized** in that, a covering (25) is provided about the keel portion (12) and spring means (14) in the shape of a foot,
said keel is rigid and contains a light-weight material therein, a top portion 38 is secured to the said opposed side walls (35, 36), and extends therebetween, said top portion including means (48, 49) for securing the artificial foot to a prosthesis, and said keel (12) provides sufficient compressive and lateral strength for the user of said foot.

2. Artificial foot as defined in claim 1 wherein said keel (12) is made from an aramid fiber-reinforced plastic material.

3. Artificial foot as set forth in claim 1 wherein the cavity of said keel (12) is filled with a foam, plastic material.

4. Artificial foot as set forth in claim 1 wherein each of the side walls of said keel (12) includes an upwardly-extending member (32), and said top portion (38) is a cover plate which includes a corresponding recess for mating with said upwardly-extending members.

5. Artificial foot as set forth in claim 1 wherein the lower wall of said keel (12) includes a first forwardly-extending portion (31), a second, intermediate, upwardly-extending portion (32) for securing said spring means (14) thereto, and a third rearwardly-extending portion (33) extending aft of said spring means (14) to define the heel portion (18) of said foot.

6. Artificial foot as set forth in claim 5 wherein a medium density, flexible foam plastic material wedge (22) is provided beneath said third portion of said keel (12).

7. Artificial foot as set forth in claim 1 wherein said spring means (14) includes a primary deflection plate (14) and an auxiliary deflection plate (20) interposed between said primary deflection plate (14) and said lower wall (30) of said keel (12).

8. Artificial foot as set forth in claim 7 wherein said auxiliary deflection plate (30) includes a forwardly-extending upwardly-oriented portion acting as a second spring during flexure of said foot.

9. Artificial foot as set forth in claim 7 wherein said primary deflection plate (14) includes at least a pair of deflection plates (14a, 14b) secured to said keel (12) and forwardly-extending therefrom in the toe portion of said keel (12), said plates being relatively slidable one with respect to the other.

10. Artificial foot as set forth in claim 7 wherein said primary deflection plate (14) is made from a composite carbon-fiber material.

11. Artificial foot as set forth in claim 7 wherein each of said primary and said secondary deflection plate (14; 20) is made from a composite carbon-fiber material.

12. Artificial foot as set forth in claim 9 further including an abrasion-resistant material interposed about said primary and secondary deflection plates (14; 20) intermediate said deflection plates and said covering to inhibit abrasion of said covering therefrom.

## Patentansprüche

1. Künstlicher Fuß, der einen Keilteil (12) hat, der einen formgepreßten Verbundkeil, der so geformt ist, daß er einen Hohlraum definiert, der eine untere Wand, ein Paar sich nach oben erstreckende einander gegenüberliegende Seitenwände (35,36), die sich mit der unteren Wand vereinigen, um einen Hohlraum dazwischen zu definieren, eine gepolsterte Ferse (22), Federmittel (14), die an dem Keil befestigt sind, um für eine Energieabsorption und -freisetzung während der Biegung des Fußes durch einen Nutzer zu sorgen, umfaßt,
dadurch gekennzeichnet, daß eine Abdeckung (25) über dem Keilteil (12) und Federmittel (14) in der Gestalt eines Fußes vorgesehen ist, wobei der Keil steif ist und leichte Materialien in demselben enthält, wobei ein Oberteil (38) an den einander gegenüberliegenden Seitenwänden (35,36) befestigt ist und sich dazwischen erstreckt, wobei dieses Oberteil Mittel (48,49) für eine Befestigung des künstlichen Fußes an einer Prothese beinhaltet und wobei der Keil (12) für ausreichende Druck- und seitliche Festigkeit für den Nutzer des Fußes sorgt.

2. Künstlicher Fuß nach Anspruch 1, wobei der Keil (12) aus einem mit Aramidfasern verstärkten Kunststoffmaterial hergestellt ist.

3. Künstlicher Fuß nach Anspruch 1, wobei der Hohlraum des Keils (12) mit einem Schaumkunststoffmaterial gefüllt ist.

4. Künstlicher Fuß nach Anspruch 1, wobei jede Seitenwand des Keils (12) ein sich nach oben erstreckendes Element (32) einschließt und wobei der obere Teil (38) eine Deckplatte ist, welche einen entsprechenden Rücksprung beinhaltet, der in die sich nach oben erstreckenden Elemente paßt.

5. Künstlicher Fuß nach Anspruch 1, wobei die untere Wand des Keils (12) einen ersten sich nach vorn erstreckenden Teil (31), einen zweiten dazwischenliegenden sich nach oben erstreckenden Teil (32) für eine Befestigung der Federmittel (14) daran 7 und einen dritten sich nach hinten erstreckenden Teil (33) beinhaltet, der sich hinter dem Federmittel (14) erstreckt, um den Fersenteil (18) des Fußes zu definieren.

6. Künstlicher Fuß nach Anspruch 5, wobei ein Keil (22) aus mitteldichtem Schaumkunststoff (22) unter dem dritten Teil des Keils (12) vorgesehen ist.

7. Künstlicher Fuß nach Anspruch 1, wobei das Federmittel (14) eine primäre Federungsplatte (14) und eine Hilfsfederungsplatte (20) beinhaltet, die zwischen der primären Federungsplatte (14) und der unteren Wand (30) des Keils (12) angeordnet ist.

8. Künstlicher Fuß nach Anspruch 7, wobei die Hilfs-Federungsplatte (30) einen sich nach vorn erstreckenden nach oben orientierten Teil hat, der als zweite Feder während der Biegung des Fußes wirkt.

9. Künstlicher Fuß nach Anspruch 7, wobei die primäre Federungsplatte (14) mindestens ein Paar Federungsplatten (14a, 14b) umfaßt, die an dem Keil (12) befestigt sind und sich von da aus nach vorn in den Zehenteil des Keils (12) erstrecken, wobei die Platten relativ zueinander verschieblich sind.

10. Künstlicher Fuß nach Anspruch 7, wobei die primäre Federungsplatte aus einem faserverstärkten Verbundkohlenstoffmaterial hergestellt ist.

11. Künstlicher Fuß nach Anspruch 7, wobei sowohl die primäre Federungsplatte, als auch die Hilfs-Federungsplatte (14,20) beide aus einem faserverstärkten Verbundkohlenstoffmaterial hergestellt sind.

12. Künstlicher Fuß nach Anspruch 9, der weiterhin ein abriebbeständiges Material einschließt, das über der primären und der Hilfs-Federungsplatte (14,20) zwischen den Federungsplatten und der Abdeckung angeordnet ist, um einen Abrieb der Abdeckung davon zu hemmen.

## Revendications

1. Pied artificiel ayant une portion de quille (12) comprenant une quille moulée composite de compression formée pour définir une cavité ayant une paroi inférieure, une paire de parois latérales opposées s'étendant vers le haut (35, 36) qui se fondent dans ladite paroi inférieure pour définir entre elles une cavité, un talon rembourré (22), un organe à ressort (14) fixé à la quille pour fournir de l'absorption d'énergie et du soulagement pendant la flexion du pied par un utilisateur, **caractérisé en ce** qu'un élément de couverture (25) se trouve autour de la portion de quille (12) et de l'organe à ressort (14) en forme de pied, que ladite quille est rigide et contient une matière légère en poids à l'intérieur, qu'une portion de dessus (38) est fixée aux parois latérales opposées mentionnées (35, 36) et s'étend entre celles-ci, ladite portion de dessus comprenant des moyens (48, 49) pour fixer le pied artificiel à une prothèse, et que ladite quille (12) fournit une résistance à la compression et latérale suffisante pour l'utilisateur du pied mentionné.

2. Pied artificiel selon la revendication 1, dans lequel ladite quille (12) est faite en une matière plastique renforcée en fibres d'aramide.

3. Pied artificiel selon la revendication 1, dans lequel la cavité de ladite quille (12) est remplie d'une matière plastique alvéolaire.

4. Pied artificiel selon la revendication 1, dans lequel chacune des parois latérales de ladite quille (12) comprend un organe qui s'étend vers le haut (32) et ladite portion de dessus (38) est une plaque de couverture qui comprend un creux correspondant pour s'accoupler avec les organes mentionnés qui s'étendent vers le haut.

5. Pied artificiel selon la revendication 1, dans lequel la portion inférieure de ladite quille (12) comprend une première portion qui s'étend vers l'avant (31), une seconde portion intermédiaire qui s'étend vers le haut (32) pour fixer l'organe à ressort mentionné (14) à celle-ci et une troisième portion qui s'étend vers l'arrière (33) qui s'étend à l'arrière de l'organe à ressort mentionné (14) pour définir la portion de talon (18) du pied mentionné.

6. Pied artificiel selon la revendication 5, dans lequel une clavette (22) de matière plastique alvéolaire flexible de densité moyenne se trouve au-dessous de ladite troisième portion de ladite quille (12).

7. Pied artificiel selon la revendication 1, dans lequel ledit organe à ressort (14) comprend une plaque de déflexion primaire (14) et une plaque de déflexion auxiliaire (20) interposée entre ladite plaque de déflexion primaire (14) et ladite paroi inférieure de ladite quille (12).

8. Pied artificiel selon la revendication 7, dans lequel ladite plaque de déflexion auxiliaire (30) comprend une portion orientée vers le haut qui s'étend vers l'avant qui agit comme un second ressort pendant la flexion du pied mentionné.

9. Pied artificiel selon la revendication 7, dans lequel ladite plaque de déflexion primaire (14) comprend au moins une paire de plaques de déflexion (14a, 14b) fixées à ladite quille (12) et qui s'étendent vers l'avant à partir de celle-ci dans la portion des orteils de ladite quille (12), les plaques mentionnées pouvant coulisser relativement l'une par rapport à l'autre.

10. Pied artificiel selon la revendication 7, dans lequel ladite plaque de déflexion primaire (14) est faite en une matière composite aux fibres de carbone.

11. Pied artificiel selon la revendication 7, dans lequel chacune des plaques de déflexion primaire et secondaire mentionnées (14 ; 20) est faite en une matière composite aux fibres de carbone.

12. Pied artificiel selon la revendication 9 comprenant de plus une matière résistante à l'abrasion interposée autour des plaques de déflexion primaire et secondaire mentionnées (14 ; 20) entre les plaques de déflexion intermédiaires mentionnées et ladite couverture pour empêcher l'abrasion de ladite couverture à partir de là.
